# EUROPÄISCHE PATENTSCHRIFT

(11) **EP 2 875 123 B1**
(45) Veröffentlichungstag und Bekanntmachung des Hinweises auf die Patenterteilung: **26.12.2018**
(21) Anmeldenummer: 13739224.7
(22) Anmeldetag: 19.07.2013
(51) Int. Cl.: C12N 5/04, C12P 21/00, C07K 1/34, C12M 1/00, C12N 1/02

(54) **FILTRATION VON ZELLKULTURÜBERSTÄNDEN**
FILTRATION OF CELL CULTURE SUPERNATANTS
FILTRATION DE SURNAGEANTS DE CULTURES CELLULAIRES

(30) Priorität: 20.07.2012 EP 12177277
(43) Veröffentlichungstag der Anmeldung: 27.05.2015
(73) Patentinhaber: Greenovation Biotech GmbH, 79108 Freiburg (DE)
(72) Erfinder: GROSSE, Thomas, 79108 Freiburg (DE); NIEDERKRÜGER, Holger, Dr., 79364 Malterdingen (DE); SCHAAF, Andreas, 79104 Freiburg (DE)
(74) Vertreter: Sonn & Partner Patentanwälte
(86) Internationale Anmeldenummer: PCT/EP2013/065264
(87) Internationale Veröffentlichungsnummer: WO 2014/013045

(56) Entgegenhaltungen:
- EP-A1- 2 853 893
- WO-A1-2005/108596
- BUETTNER-MAINIK ANNETTE ET AL: "Production of biologically active recombinant human factor H in Physcomitrella", PLANT BIOTECHNOLOGY JOURNAL, Bd. 9, Nr. 3, April 2011 (2011-04), Seiten 373-383, XP002688821,
- LUCUMI ET AL: "Establishment of long-term perfusion cultures of recombinant moss in a pilot tubular photobioreactor", PROCESS BIOCHEMISTRY, ELSEVIER, NL, Bd. 41, Nr. 10, 1. Oktober 2006 (2006-10-01), Seiten 2180-2187, XP005655505, ISSN: 1359-5113, DOI: 10.1016/J.PROCBIO.2006.06.017
- Marta Fernandez Nunez: "Cytokinin profiling beim Laubmoos Physcomitrella patens (Hedw.) B.S.G.- Einflüsse von Cytokinin-Oxidase/Dehydrogenase sowie Adenosin-Kinase", Bibliothekssystem Universität Hamburg - E-Dissertationen der Universität Hamburg , 30. Oktober 2010 (2010-10-30), Seiten FRONTPG.-162, XP002688814, Gefunden im Internet: URL:http://ediss.sub.uni-hamburg.de/vollte xte/2010/4787/ [gefunden am 2012-12-07]
- FISCHER R ET AL: "Affinity-purification of a TMV-specific recombinant full-size antibody from a transgenic tobacco suspension culture", JOURNAL OF IMMUNOLOGICAL METHODS, ELSEVIER SCIENCE PUBLISHERS B.V.,AMSTERDAM, NL, Bd. 226, Nr. 1-2, 24. Juni 1999 (1999-06-24), Seiten 1-10, XP002360521, ISSN: 0022-1759, DOI: 10.1016/S0022-1759(99)00058-7
- KIM SUN TAE ET AL: "Secretome analysis of differentially induced proteins in rice suspension-cultured cells triggered by rice blast fungus and elicitor", PROTEOMICS, Bd. 9, Nr. 5, März 2009 (2009-03), Seiten 1302-1313, XP002688822, ISSN: 1615-9853
- NDIMBA BONGANI K ET AL: "Proteomic analysis of changes in the extracellular matrix of Arabidopsis cell suspension cultures induced by fungal elicitors.", PROTEOMICS, Bd. 3, Nr. 6, Juni 2003 (2003-06), Seiten 1047-1059, XP002688823, ISSN: 1615-9853
- DANQUAH M K ET AL: "Dewatering of microalgal culture for biodiesel production: Exploring polymer flocculation and tangential flow filtration", JOURNAL OF CHEMICAL TECHNOLOGY AND BIOTECHNOLOGY 2009 JOHN WILEY AND SONS LTD GBR, vol. 84, no. 7, 2009, pages 1078-1083, ISSN: 0268-2575
- MOLINA GRIMA E ET AL: "Recovery of microalgal biomass and metabolites: process options and economics", BIOTECHNOLOGY ADVANCES, ELSEVIER PUBLISHING, BARKING, GB, vol. 20, no. 7-8, 1 January 2003 (2003-01-01), pages 491-515, XP004400158, ISSN: 0734-9750, DOI: 10.1016/S0734-9750(02)00050-2
- SELLSTEDT A: "OCCURRENCE AND ACTIVITY OF HYDROGENASE IN SYMBIOTIC FRANKIA FROM FIELD-COLLECTED ALNUS-INCANA", PHYSIOLOGIA PLANTARUM, MUNKSGAARD INTERNATIONAL PUBLISHERS, COPENHAGEN, DK, vol. 75, no. 2, 1 January 1989 (1989-01-01), pages 304-308, XP009502721, ISSN: 0031-9317, DOI: 10.1111/J.1399-3054.1989.TB06186.X

## Beschreibung

Die vorliegende Erfindung betrifft Verfahren zur Filtration von Zellkulturüberständen.

Die Verwendung biotechnologischer Verfahren zu Produktionszwecken stellt eine bedeutende Möglichkeit dar, Substanzen zu produzieren, die auf anderem Weg, z.B. durch chemische Synthese, nicht bzw. nicht wirtschaftlich herzustellen sind und als Rohstoffe in der Natur nicht in ausreichenden Mengen zur Verfügung stehen. Die Produktion von pflanzlichen Stoffen konzentriert sich derzeit auf Sekundärmetabolite, wobei der Anteil an rekombinanten Expressionsprodukten stetig steigt. Die großtechnische Produktion konzentriert sich vor allem auf pflanzliche Zellkulturen oder Pflanzen. Durch die Möglichkeit des DNA-Transfers in Pflanzen wurden nicht nur quantitative und qualitative Veränderungen von Pflanzeninhaltsstoffen möglich. Pflanzen und pflanzliche Zellkulturen wurden darüber hinaus für die Herstellung heterologer Proteine interessant (Fischer et al., Current Opinion in Plant Biology 2004, 7: 1-7).

Der eine Ansatz beinhaltet die Produktion heterologer Proteine in transgenen vollständigen Pflanzen. Ein grundsätzlicher Nachteil bei der Verwendung vollständiger Pflanzen wie der oben beispielhaft beschriebenen liegt in der Notwendigkeit ihrer zeitaufwendigen und kostenintensiven Kultivierung sowie in der für industrielle Produktionsmaßstäbe erforderlichen großdimensionierten Anbaufläche. Darüber hinaus erfordert die Aufreinigung der gewünschten Zielsubstanzen aus vollständigen Pflanzen in der Regel komplexe Verfahrensschritte, insbesondere dann, wenn an die Beschaffenheit und Qualität der Produkte hohe Anforderungen gestellt werden, wie es bei pharmazeutisch oder ernährungsphysiologisch einzusetzenden Substanzen der Fall ist.

Im zweiten Ansatz wurden transgene Pflanzenzellkulturen für die Produktion von Antikörpern genutzt. Bekannt ist beispielsweise die Expression von Antikörpern oder anderer Säugetierproteine (Huether et al., Plant Biol. 2005, 7: 292-299) und deren Sekretion ins Medium. Da die Aufreinigung heterologer Proteine aus Zellen einen hohen Aufwand erfordert, stellt die Sekretion des Zielproteins in das Medium eine deutliche Verbesserung dar. Darüber hinaus sprechen auch Sicherheitsaspekte für die Produktion rekombinanter, pharmazeutisch relevanter Proteine in Zellkulturen, da die transgenen Pflanzenzellen ausschließlich in Bioreaktoren kultiviert werden können und nicht freigesetzt werden müssen. Die Entwicklung von Bioreaktoren für heterotrophe pflanzliche Zellkulturen in größeren Maßstäben machte die notwendige Massenkultur möglich.

Für eine weitere Produktaufreinigung muss in einem ersten Schritt die Biomasse vom Überstand getrennt werden. Hierzu sind verschiedene Filtrationsprozesse bekannt. Im Fall von rekombinanten sekretierten Proteinen liegt das Produkt im Kulturüberstand vor. Das Ziel liegt daher darin, möglichst wenig Verbleib von Restfeuchte/Überstand in der abgetrennten Biomassefraktion zurückzuhalten, um Produktverluste zu vermeiden. Diverse Filtrationsmethoden im Labormaßstab sind bekannt. Buettner-Mainik Annette et al. (Plant Biotechnology Journal 9 (3) (2011): 373-383) beschreibt die Produktion von menschlichem Faktor H in *P*. *patens.* Wässrige Zellkultur wird durch ein Gewebe über einer Nutsche unter Vakuum abgetrennt. Das erhaltene Produkt war feucht und musste getrocknet werden. Lucumi et al. (Process Biochemistry 41 (10) (2006): 2180-2187) beschreibt eine Querstromfiltration während der Herstellung von rh VGEF in einem *P. patens* Bioreaktor. Marta Fernandez Nunez (E-Dissertation der Universität Hamburg (2010), S. 48, Kapitel 2.11.1) beschreibt eine Vakuumfiltration beim Cytokinin-Profiling in *P. patens.* Fischer Rainer et al. (Journal of Immunological Methods 226 (1-3) (1999): 1-10) betrifft eine Vakuumfiltration von Tabak Callus Kulturen, welche nach der Filtration feucht waren. WO 2005/108596 A1 betrifft die *in vitro* Synthese von zyklischen Peptiden mit einem Cysteinknoten durch Expression in Pflanzenzellen. Zellsuspensionen wurden mit einer Nutsche unter Vakuum abgetrennt. Kim Sun Tae et al. (Proteomics 9 (5) (2009): 1302-1313) beschreibt eine Cokultur von Reiszellen mit dem Pilz *M*. *grisea* in Suspension. Eine Abtrennung von sekretierten Proteinen erfolgte mittels Vakuumfiltration. Ndimba Bongani K. (Proteomics 3 (6) (2003): 1047-1059) behandelt eine Untersuchung zu Änderungen der extrazellulären Matrix von Arabidopsis in Suspensionskulturen. Zur Untersuchung des Kulturüberstandes wurde eine Vakuumfiltration eingesetzt.

Eine spezielle Problematik liegt darin, dass bei Pflanzen und Pflanzenzellkulturen im Gegensatz zu Prozessen mit tierischen Zellen und Bakterien der feste Rückstand, z.B. der Filterkuchen im Fall der Filtration, ein immenses Volumen einnimmt. Dadurch werden große Mengen des Überstandes gebunden und zurückgehalten. Ein Ziel der vorliegenden Erfindung liegt daher in einer verbesserten Abtrennung von fester Biomasse und dem Kulturüberstand.

Die vorliegende Erfindung betrifft ein Verfahren zur Abtrennung eines flüssigen Überstandes von Zellen mit einer Zellwand umfassend die Schritte:
a) Bereitstellen einer Mischung der Zellen mit einer Flüssigkeit,
b) Befüllen eines Filtergehäuses mit der Mischung, wobei im Filtergehäuse ein erster Filter mit einer Porengröße von zwischen 4 µm bis 50 µm auf einer flachen Bodenfläche vorgesehen ist und die Wände des Filtergehäuses dicht mit der flachen siebartig durchbrochenen Bodenfläche verbunden sind,
c) Ansetzen eines Differenzdrucks von mindestens 0,5 bar (500 hPa) an der Mischung durch Druckluft, wodurch der Flüssigkeitsanteil der Mischung durch den Filter gepresst wird und ein Filterkuchen enthaltend Zellen im Filtergehäuse zurückbleibt, wobei der absolute Druck an der Entnahmestelle mindestens 0,7 bar ist,
d) Entnahme der filtrierten Flüssigkeit.
Eine Filtration mit dem erfindungsgemäßen Filter würde im Grunde auch ohne die Verwendung eines flachen Bodenfilters (z.B. mit einem Beutelfilter) und auch ohne Druck erfolgreich ablaufen, d.h. eine rein durch Gravitation getriebene Filtration. Eine derartige Filtration leidet jedoch unter dem Nachteil, dass eine hohe Restfeuchte im Filterkuchen zurückbleibt. Sofern das gewünschte Reinigungsprodukt in der Flüssigkeit vorliegt, bedeutet dies einen hohen Produktverlust.

Die Bodenfläche ist vorzugsweise siebartig durchbrochen. Die Bodenfläche kann selbst den Filter darstellen oder sie kann als Auflage und Stützung eines separaten Filters dienen.

Erfindungsgemäß konnte durch Einsatz des flachen Filters auf einer bestimmten Bodenfläche in Kombination mit Druckerhöhung überraschenderweise die im Filterkuchen verbliebene Restfeuchte auf ein unmerkliches Mindestmaß reduziert werden. Extrazelluläre Restfeuchten von 0 % (Gew.-%) konnten erzielt werden. Die intrazelluläre Flüssigkeit wird hierbei nicht zur Restfeuchte gezählt. Erfindungsgemäß wurde die Isolierung des Überstandes ohne intrazellulärer Flüssigkeit verbessert, da die intrazelluläre Restfeuchte keinen Produktverlust für sezernierte Proteine darstellt, und zudem der intrazelluläre Zellbestand eine kontaminierende Mischung darstellen kann.

Erfindungsgemäß wird in vorzugsweisen Ausführungsformen der Differenzdruck solange aufrechterhalten, bis im Filterkuchen eine extrazelluläre Restfeuchte von unter 3 % (Gew.-%), vorzugsweise von unter 2 %, speziell bevorzugt von unter 1 %, oder von 0%, erreicht wird. Die Restfeuchte kann wie in Vergleichsbeispiel 1 bestimmt werden. Insbesondere kann das erfindungsgemäße Filtergehäuse mit dem genannten Filter verwendet werden, um Feuchtigkeit aus einem Filtrierkuchen von pflanzlichen Zellen zu entfernen - insbesondere bis zu den genannten Restfeuchten.

In bevorzugten Ausführungsformen ist der Filter eine Flachmembran. Dies sind z.B. Vliese, keramische Gießlinge oder Polymermembranen, die in flacher Form verwendet werden. Sie spielen in großtechnischen Anwendungen üblicherweise kaum eine Rolle. Erfindungsgemäß hat sich gezeigt, dass in der Pflanzenzellfiltration flache Filter dennoch sehr effektiv eingesetzt werden können, insbesondere bei der Druckfiltration, und sogar geringere Restfeuchten ermöglicht, als beispielsweise die üblicheren Taschen- oder Sackfilter.

Das erfindungsgemäße Filtriersystem für die erfindungsgemäßen Verfahren wurde insbesondere für die großtechnische Anwendung entwickelt. Somit sind die Dimensionen des Filtergehäuses in vorzugsweisen Ausführungsformen für die Filtrierung großer Flüssigkeitsvolumen ausgelegt. Insbesondere bevorzugt ist das Innenvolumen des Filtergehäuses mindestens 2 l (dm³), im speziellen mindestens 5 l, mindestens 8 l, mindestens 10 l, mindestens 20 l, mindestens 30 l, mindestens 40 l oder mindestens 45 l.

Der Innenraum des Gehäuses kann kontinuierlich oder diskontinuierlich mit weiterem Überstand zur Filtrierung befüllt werden. In vorzugsweisen Ausführungsformen werden mindestens 2 l (dm³) Überstand filtriert, in speziellen Ausführungsformen werden mindestens 5 l, mindestens 8 l, mindestens 10 l, mindestens 20 l, mindestens 40 l, mindestens 50 l, mindestens 75 l oder mindestens 100 l, filtriert.

Vorzugsweise werden die Zellen während des Verfahrens nicht aufgebrochen. Dies kann mittels schonender Behandlung, bei geringen Drücken, erreicht werden. Die Vermeidung des Aufbrechens der Zellen ist mit dem Vorteil verbunden, dass die Zellinhalte das Filtrationsprodukt nicht verunreinigen und weiters, dass die Zellen besser abfiltriert werden können.

Der absolute Druck an der Entnahmestelle des filtrierten Produkts ist mindestens 0,7 bar (700 hPa). Erfindungsgemäß wird keine Vakuumfiltration vorgenommen, sondern das Filtrat bleibt bei hohen Atmosphärendrücken von mindestens 0,7 bar, vorzugsweise mindestens 0,8 bar, mindestens 0,9 bar oder mindestens 1 bar. Dadurch wird ein Verdampfen des flüssigen (insbesondere wässrigen) Filtrats vermieden. D.h. die erfindungsgemäße Druckerhöhung im Filtergehäuse bzw. an der Mischung, speziell am Einlass des Filtergehäuses, führt zu einem absoluten Überdruck, d.h. einem Druck größer als der Umgebungsdruck.

Der erfindungsgemäße Überdruck (Differenzdruck zwischen befülltem Überstand und filtrierter Flüssigkeit) kann auf verschiedene Arten erzeugt werden. Es wird durch Druckluft (z.B. zugeführt am Einlass in das Filtergehäuse oder an einer separaten Luftzufuhröffnung) der Druck hergestellt. Zusätzlich kann Druck durch einen hydraulischen Druck, z.B. als Flüssigkeitsstaudruck oder über eine Pumpe hergestellt werden. Eine weitere Möglichkeit ist die Druckerhöhung durch Zentrifugation. Auch möglich ist ein mechanischer Druck, z.B. über einen Stempel. Selbstverständlich können alle diese Alternativen auch miteinander kombiniert werden. Vorzugsweise wird zunächst die Mischung der Zellen mit der Flüssigkeit unter Druck zugeführt und anschließend nach Einbringen der gesamten zu filtrierenden Mischung weitere Flüssigkeit über Druckluft ausgebracht. Die Druckluft kann bei den gleichen Drücken angelegt werden - identisch oder unabhängig ausgewählt vom Flüssigkeitsdruck.

In vorzugsweisen Ausführungsformen ist der Differenzdruck mindestens 0,8 bar (800 hPa), vorzugsweise mindestens 1 bar (1000 hPa), oder auch mindestens 1,2 bar (1200 hPa). In speziellen Ausführungsformen ist der Druck maximal 8 bar, maximal 5 bar, maximal 3 bar, maximal 2 bar oder maximal 1,5 bar. Die Druckdifferenz wird maßgeblich durch den verwendeten Filter (Porengröße, Dimension) bestimmt, wodurch ein gewisser Druckwiderstand bei einem gewählten Überdruck am Einlass des Filtergehäuses entsteht. Der gewählte Überdruck kann beispielsweise bei geschlossenem Auslass gemessen werden und ist vorzugsweise ein absoluter Druck von mindestens 1,8 bar (1800 hPa), vorzugsweise mindestens 2 bar (2000 hPa), oder auch mindestens 3 bar (3000 hPa).

Daher ist vorzugsweise das Filtergehäuse ein Druckgefäß, insbesondere ein Druckgefäß ausgelegt für Drücke von mindestens 2 bar (2000 hPa), im speziellen bevorzugt von mindestens 5 bar (5000 hPa) oder auch von mindestens 8 bar (8000 hPa).

Die Porengröße des ersten Filters ist zwischen 4 µm und 50 µm. In bevorzugten Ausführungsformen ist die Porengröße des erster Filters zwischen 6 µm und 40 µm oder zwischen 8 µm und 30 µm, insbesondere zwischen 9 µm und 250 µm.

Die siebartig durchbrochene Bodenfläche ist erfindungsgemäß flach und ohne Wölbung oder kaum gewölbt. Vorzugsweise sind die siebartigen Löcher ringförmig angeordnet. Sie sind vorzugsweise mindestens 2 cm vom Rand der Bodenfläche, der mit der Gefäßwand dicht verbunden ist, entfernt. Die siebartigen Löcher können Größen von 0,001 mm bis 5 mm, vorzugsweise von 0,005 mm bis 3 mm oder von 0,01 mm bis 1,5 mm, am meisten bevorzugt von 0,03 mm bis 0,06 mm, aufweisen.

Die Temperatur während des Verfahrens liegt vorzugsweise zwischen 0 °C und 40 °C, insbesondre zwischen 10 °C und 30 °C, für eine schonende Filtration der Zellen.

Erfindungsgemäß ist es aufgrund der effizienten Reduzierung der Restfeuchte im Filterkuchen nicht notwendig, diesen zusätzlich zu waschen. Obwohl dieser Schritt selbstverständlich vorgenommen werden kann, wird in vorzugsweisen Ausführungsformen der Filterkuchen nicht durch zusätzliche Flüssigkeit gewaschen. In anderen Ausführungsformen werden nur geringe Volumina Waschflüssigkeit verwendet, z.B. maximal 1 % oder maximal 0,1 % des Volumens des Überstandes und/oder maximal 5 % oder maximal 2 % des Volumens des Filterkuchens.

In besonders bevorzugten Ausführungsformen folgt der oben gennannten Filtration eine weitere Filtration, insbesondere eine Tiefenfiltration und/oder eine Sterilfiltration.

Vorzugsweise umfasst das erfindungsgemäße Verfahren weiters den Schritt: e1) weiteres Filtrieren der in Schritt d) erhaltenen filtrierten Flüssigkeit mit einem zweiten Filter mit einer geringeren Porengröße als der erste Filter, wobei die Porengröße des zweiten Filters im Bereich von 1 µm bis 20 µm ist. Vorzugsweise ist die Porengröße des zweiten Filters im Bereich von 2 µm bis 15 µm, insbesondere im Bereich von 3 µm bis 10 µm, im speziellen von 4 µm bis 8 µm.

Vorzugsweise umfasst das erfindungsgemäße Verfahren weiters den Schritt: e2) weiteres Filtrieren der in Schritt d) oder e1) erhaltenen filtrierten Flüssigkeit mit einem dritten Filter mit einer geringeren Porengröße als der zweite Filter, wobei die Porengröße des dritten Filters im Bereich von 0,25 µm bis 10 µm ist. Vorzugsweise ist die Porengröße des dritten Filters im Bereich von 0,3 µm bis 5 µm, insbesondere im Bereich von 0,35 µm bis 2 µm, im speziellen von 0,4 µm bis 1,5 µm.

Vorzugsweise umfasst das erfindungsgemäße Verfahren weiters den Schritt: f) weiteres Filtrieren der in Schritt e1) oder e2) erhaltenen filtrierten Flüssigkeit mit einem vierten Filter mit einer geringeren Porengröße als der dritte Filter, wobei die Porengröße des dritten Filters im Bereich von 0,05 µm bis 2 µm ist. Vorzugsweise ist die Porengröße des vierten Filters im Bereich von 0,08 µm bis 1,5 µm, insbesondere im Bereich von 0,1 µm bis 1 µm, im speziellen von 0,15 µm bis 0,6 µm oder bis 0,4 µm.

Die Angaben "erster", "zweiter", "dritter" und "vierter" Filter bedeutet nicht, dass das erfindungsgemäße Verfahren auf diese Anzahl an Filtern limitiert ist oder dass exakt diese Anzahl zum Einsatz an Filtern kommt. Sie dient lediglich zur Unterscheidung verschiedener Filter mit unterschiedlichen Porengrößen. Vorzugsweise werden im erfindungsgemäßen Verfahren 1, 2, 3, 4 oder mehr Filtrierungsschritte unternommen, wobei in vorzugsweisen Ausführungsformen die Filter in nachfolgenden Schritten eine kleinere Porengröße aufweisen als die Filter im vorhergehenden Schritt.

Als Filter-Materialien für den ersten, zweiten, dritten und/oder vierten Filter kommen beliebige Filter, welche zur Zellfiltrierung geeignet sind, in Frage. Beispielsmaterialen sind z.B. aus Zellulose oder Polymermembranen. Beispiele sind Polyethersulfon-Membranen (wie z.B. Sartopore-Filter), oder Zellulose/Kieselgur/Perlit-Filter (wie z.B. Seitz K-Serien Filter).

Die im erfindungsgemäßen Verfahren einzusetzende Zelle ist vorzugsweise eine pflanzliche Zelle, vorzugsweise eines Mooses, insbesondere ausgewählt aus der Gruppe bestehend aus Laubmoosen und Lebermoosen, wobei Spezies aus den Gattungen Physcomitrella, Funaria, Sphagnum und Ceratodon, bzw. Marchantia und Sphaerocarpos besonders bevorzugt eingesetzt werden. Besonders bevorzugt ist *Physcomitrella patens.* Am meisten bevorzugt wird das erfindungsgemäße Verfahren unter Verwendung von Zellen, Pflanzen oder Pflanzengewebe wie Protonema des Laubmooses Physcomitrella patens durchgeführt. Weitere bevorzugte Pflanzen sind Tabak, Bohnen oder Linsen. Bevorzugt ist die Pflanze eine Wasserpflanze, z.B. der Gattungen Lemna, Spirodela, Landoltia, Wolffia oder Wolffiella.

Das erfindungsgemäße Verfahren ist insbesondere geeignet, die Zellen mit einer Zellwand zu filtrieren. Neben echten Pflanzenzellen können die Zellen auch aus einer Pilzzelle oder einer Algenzelle ausgewählt sein, welche als äquivalente Ausführungsformen angesehen werden. "Zelle" wie hierin verwendet, kann isolierte Zellen, vereinzelte Zellen oder eine Zelle in einem mehrzelligen Organismus betreffen. Eine pflanzliche Zelle kann eine pflanzliche Einzelzelle oder eine Zelle in einer Pflanze oder in einem Pflanzengewebe sein. In analoger Weise kann eine Pilzzelle eine Einzelzelle oder eine Zelle in einem Pilz oder einem Pilzgewebe sein. Die Algenzelle ist vorzugsweise eine Grünalgenzelle. Gewebe können z.B. ausgewählt sein aus Phloem, Xylem, Mesophyll, Stamm, Blätter, Thallus, Protonema, Chloronema, Caulonema, Rhizoiden oder Gametophoren. Die Zellen können Protoplasten oder Parenchymzellen, insbesondere Callus-Zellen, umfassen oder daraus bestehen.

Weitere geeignete Zellen sind Bakterienzellen sofern diese eine Zellwand aufweisen. Obwohl das erfindungsgemäße zunächst für Pflanzenzellen optimiert wurde, konnten die gleichen Vorteile, wie schonende Behandlung der Zellen bei hohem Flüssigkeitsabtrennungsgrad, auch bei Bakterienzellen beobachtet werden.

Vorzugsweise ist im flüssigen Überstand ein von den pflanzlichen Zellen produziertes rekombinantes Protein. Ebenso oder in Kombination hiermit ist es möglich, dass mit der Filtrierung der Flüssigkeit ein rekombinantes Protein des Überstandes gereinigt wird. Die rekombinante Produktion von Proteinen in Pflanzen ist hinlänglich wie einleitend erwähnt bekannt und die üblichen Verfahren zur Transformation und Expression in Pflanzen können angewendet werden.

Die vorliegende Erfindung betrifft weiters Verwendung eines Kits mit einem Filtergehäuse wie hierin beschrieben und mit einem Innenvolumen von mindestens 2 L, wobei das Filtergehäuse ein Druckgefäß mit einem Einlass und einer Luftzufuhröffnung ist, und einen erster Filter wie hierin beschrieben, z.B. mit einer Porengröße von zwischen 4 µm bis 50 µm, im erfindungsgemäßen Verfahren. Der Kit oder das System kann vorzugsweise zusätzlich mit einem zweiten, dritten und/oder vierten Filter wie hierin beschrieben ausgestattet sein. Dieser Kit oder das System eignet sich zur Durchführung der erfindungsgemäßen Verfahren oder wird hierzu verwendet.

Die Erfindung betrifft auch die Verwendung des erfindungsgemäßen Kits bzw. seiner Bestandteile, um Feuchtigkeit aus einem Filtrierkuchen aus (pflanzlichen) Zellen zu entfernen.

Die vorliegende Erfindung wird weiters durch die folgenden Figuren und Beispiele illustriert, ohne auf diese speziellen Ausführungsformen der Erfindung limitiert zu sein.

### Figuren:

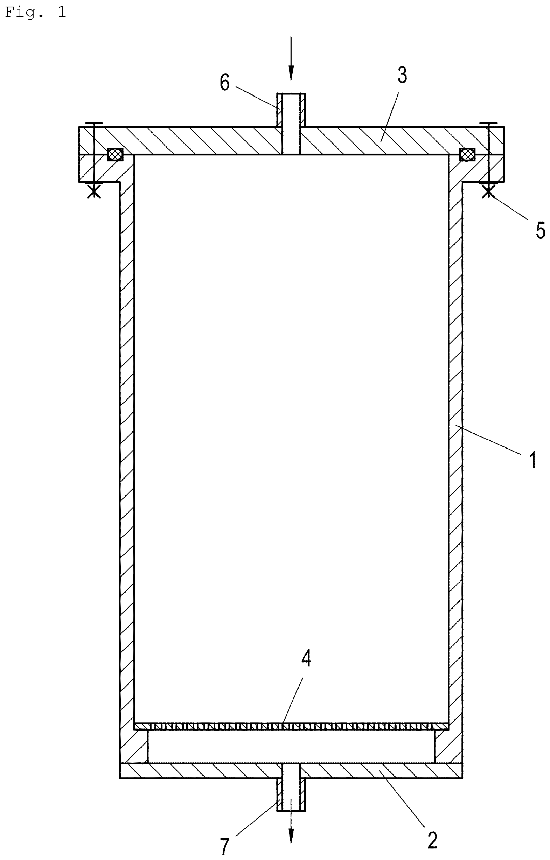

Figur 1 zeigt einen Querschnitt durch ein erfindungsgemäßes Filtergehäuse mit einer Bodenplatte (2), einem Deckel (3) und einer siebartigen Bodenfläche (4) des Befüllungsraumes als Auflage für ein Filtermaterial. Der Deckel wird über eine lösbare Schraubverbindung (5) mit der zylindrischen Gehäusewand (1) druckdicht verschlossen. Das Gehäuse verfügt weiters über einen Einlass (6) und einen Auslass (7).

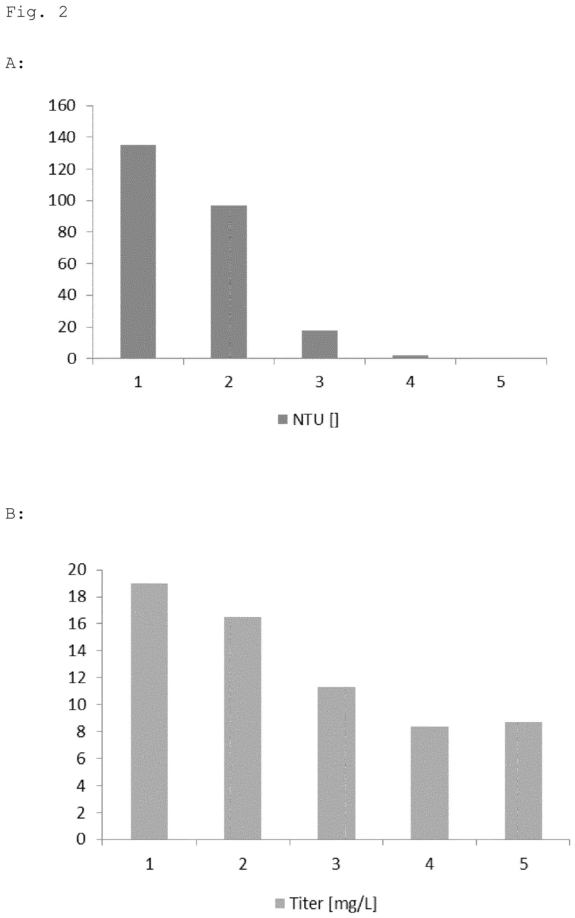

Fig. 2 zeigt die Analyseergebnisse nach der Schwebstoffreinigung über Trübungsmessungen (NTU [-]; A) sowie die Produktverluste (Titer [mg/L]; B) mit dem Supradisc-System während der Tiefenfiltration. (1 = Kulturüberstand, 2 = Moos-freies K900-Filtrat, 3 = K700-Filtrat, 4 = KS50-Filtrat, 5 = Sterilfiltrat)

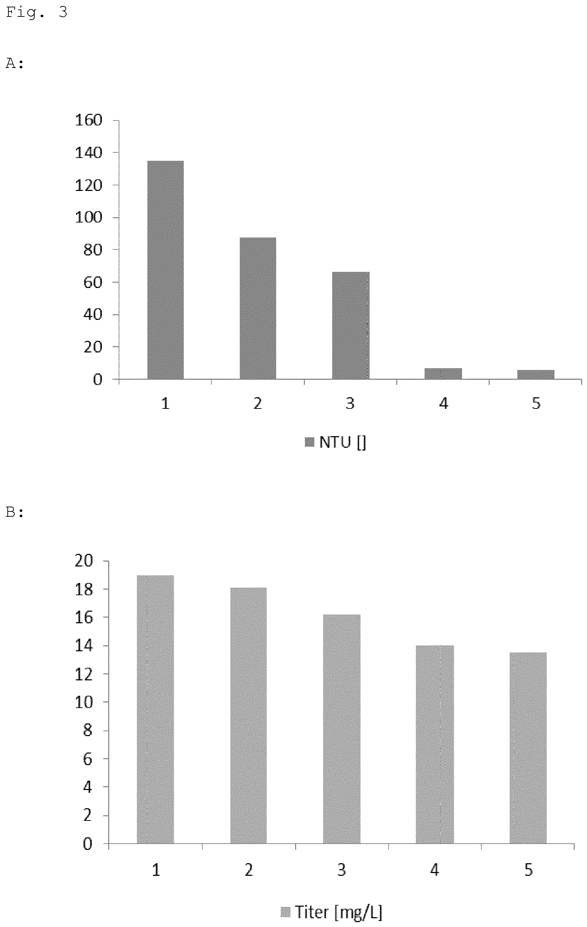

Fig. 3 zeigt die Analyseergebnisse nach der Schwebstoffreinigung über Trübungsmessungen (NTU [-]; A) sowie die Produktverluste (Titer [mg/L]; B) mit dem Supracap-System während der Tiefenfiltration. (1 = Kulturüberstand, 2 = Moos-freies K900-Filtrat, 3 = K700-Filtrat, 4 = KS50-Filtrat, 5 = Sterilfiltrat)

### Beispiele:

### Vergleichsbeispiel 1:

In kleinen Maßstäben wurde ein Überstand mit rekombinant produziertem Antikörper (carcinoembryonic antigen (CEA) specific IgG1 H10) von den produzierenden Protonema Moosgeweben, Zelldichte bis 7 g/L, abgetrennt. Hierbei wurde ein Sieb mit Porengröße 100 µm verwendet, wobei die verbleibende Restfeuchte ca. 50 % betrug.

Beim Upscale der Produktion wurde nach geeigneten Filtermethoden gesucht. Dabei sollte der erste Filtrationsschritt über einen relativ groben Filter erfolgen. Dies vermeidet eine Verblockung des Filters und dient hauptsächlich der Trennung von Moos und Überstand. Tiefenfiltrationseffekte während der Klärfiltration treten in den Hintergrund.

In einem ersten Infiltrationsschritt wurde eine Beutelfiltration vorgenommen (Firma: Eaton; Gehäuse Typ: TBF-0101-AD10-050D, Beutelfilter Artikel-Nr.: F5869549; Volumen 13 l). Der Filter lagerte in einem Gehäuseeinsatz mit einer gekrümmten Bodenfläche, wobei an der Bodenfläche und an der Einsatzwand der Einsatz siebartig durchbrochen war. Die Filtration war erfolgreich, jedoch war die Restfeuchte im abgetrennten Moos sehr hoch und konnte nicht über Druckluft (1-3 bar) aus dem Moos gedrückt werden.

Die extrazelluläre Restfeuchte wurde nach Beendigung der Filtration durch Entnahme des Filterkuchens und manuelles Auspressen desselben bestimmt. Ein volumetrischer Vergleich der ausgepressten Flüssigkeit und dem zurückbehaltenen Moosmaterial ergab ein Verhältnis von ca. 50 %.Damit einher gingen nicht separat gemessene hohe Produktverluste.

### Vergleichsbeispiel 2:

Es wurde analog zu Vergleichsbeispiel 1 in einem Upscale-Versuch Überstand mit rekombinant produziertem Antikörper von produzierenden Moosgeweben durch Filtration getrennt. Es wurde ein alternativer Gehäuseeinsatz mit einer gekrümmten Bodenfläche verwendet, wobei diesmal nur an der Bodenfläche der Einsatz siebartig durchbrochen war. Damit sollte gewährleistet werden, dass die Druckluft homogen über den Filterkuchen entweicht und die Restfeuchte aus dem Moosrückstand entfernt. Dies war jedoch nicht der Fall. Die Restfeuchte wurde wie in Vergleichsbeispiel 1 bestimmt und betrug ebenfalls ca. 50 % (Gew.-%).

### Beispiel 1: Großvolumenfiltration

Es wurde ein neues Filtergehäuse entwickelt, welches weniger Restfeuchte in abgetrennten Pflanzenzellen aufweisen soll. Das neue Gehäuse ist in Figur 1 dargestellt. Das Filtergehäuse zeichnet sich durch einen siebartigen Einsatz (4) als flache Auflage für ein Filtermaterial aus. Die Gehäusewand dient nicht als Filterfläche. Sämtliches filtriertes Fluid muss die flache Bodenplatte auf dem Weg zum Auslass passieren. Im Fall von tierischen Zellen würde dieser Filter aufgrund der geringen Filteroberfläche rasch zur Verstopfung neigen. Im Fall von Pflanzenzellen hat sich jedoch herausgestellt, dass damit nicht nur die Filtration mit großen Volumen (> 5 l) möglich ist, sondern sogar überraschenderweise den Vorteil der niedrigen Restfeuchte bietet.

Bestückt wird das Gehäuse mit einer Filterscheibe Seitz K900 (Porengröße 8-20 µm; Fa. Pall). Das Gehäuse ist für den Technikumsmaßstab bis 500 l ausgelegt worden und ist für beliebige Volumina Kulturüberstand skalierbar. Die Tests mit diesem Filtergehäuse lieferten gute Daten. Ein Testlauf mit 500 l Kulturbrühe und geringer Biomasse (0,5 g/L) sowie mehrere Versuche mit 100 l Kulturbrühe mit hoher Biomasse (4 bis 7 g/L) konnte eine extrazelluläre Restfeuchte von 0 % (bestimmt wie in Vergleichsbeispiel 1) im Filterkuchen erzielen. Dies bedeutet, dass der Überstand der Kulturbrühe in diesem Modul nacheinander über den kompletten Filterkuchen, den Filter und aus dem Filtergehäuse gedrückt wird. Die Produktverluste eines Antikörpers in mg/L fielen ebenfalls sehr gering aus (4 bis 13 % - Schritt 1 der Tabelle 1). Die Verluste entstehen hier einzig durch unspezifischen Verbleib von Produkt im Mooskuchen bzw. Filter. Nicht jedoch durch Verbleib von Kulturüberstand im Filterkuchen.

### Beispiel 2: Tiefen- und Sterilfiltration

Zur Tiefen- und Sterilfiltration wurden das Filtrat nach Beispiel 1 weiters filtriert: zur Tiefenfiltration wurde mit einer Filterscheibe Seitz K700 (Porengröße 6-12 µm; Fa. Pall) und einer Filterscheibe Seitz KS50 (Porengröße 0,4-1 µm; Fa. Pall) filtriert. Diese Filtrationen können in einem Gehäuse und in einem Durchgang sequentiell durchgeführt oder separat in getrennten Gehäusen durchgeführt werden. Die Beabstandung der Filter wurde so gewählt, dass ausreichend Platz für Mooszellen oder Zellfragmente ist. Die Filter wurden in zwei parallelen Läufen gemäß dem SUPRAdisc™ (Fa. Pall) und dem Supracap™ 100 (Fa. Pall) Systemen montiert (Supradisc-Filter: 300P700S205SP und 200P050S205SP; Supracap-Filter: NP6P7001 und NP6P0501).

Zur Sterilfiltration wurde das Filtrat weiters mit einer Filterscheibe Sartopore 2 (Porengröße 0,2 µm; Fa. Sartorius) filtriert.

### Beispiel 3: Resultate

Mit dem entwickelten Filtergehäuse kann eine optimale Fest-Flüssig-Trennung gewährleistet werden. Es bildet sich ein fester Filterkuchen aus, der weitgehend frei von Restfeuchte ist und daher den Produktverlust gering hält. Der Gesamtprozess gewährleistet, dass die weiteren Aufreinigungsschritte zügig ohne Verstopfung der Filter ablaufen können.

Die Figuren 2 und 3 zeigen die Ergebnisse. Gezeigt wird die Analytik der Reinigung von Schwebstoffen durch die Filtration durch Trübungsmessungen (NTU) und die Entwicklung der Antikörperverluste über die Filtration, jeweils für die Aufreinigung über das Supradisc-System (Fig. 2) und die Aufreinigung über Supracap-System (Fig. 3) während der Tiefenfiltration. Tabelle 1 fasst die Produktverluste zusammen.

**Tab. 1: Produktverluste bei der Aufreinigung über das Supradisc- und das Supracap-System.**

| Filtrationsschritt | Supradisc Ausbeute [%] | Verlust [%] | Supracap Ausbeute [%] | Verlust [%] |
|---|---|---|---|---|
| 1: K900 | 86,84 | -13,15 | 95,26 | -4,73 |
| 2: K700 | 68,48 | -31,51 | 89,50 | -10,49 |
| 3: KS50 | 74,33 | -25,66 | 86,41 | -13,58 |
| 4: Sterilfiltr. | 103,57 | 3,57 | 96,42 | -3,57 |
| Gesamt | 45,78 | -54,21 | 71,05 | -28,94 |

## Patentansprüche

1. Verfahren zur Abtrennung eines flüssigen Überstandes von Zellen mit einer Zellwand umfassend die Schritte:
a) Bereitstellen einer Mischung der Zellen mit einer Flüssigkeit,
b) Befüllen eines Filtergehäuses mit der Mischung, wobei im Filtergehäuse ein erster Filter mit einer Porengröße von zwischen 4 µm bis 50 µm auf einer flachen Bodenfläche vorgesehen ist und die Wände des Filtergehäuses dicht mit der flachen siebartig durchbrochenen Bodenfläche verbunden sind,
c) Ansetzen eines Differenzdrucks von mindestens 0,5 bar an der Mischung durch Druckluft, wodurch der Flüssigkeitsanteil der Mischung durch den Filter gepresst wird und ein Filterkuchen enthaltend Zellen im Filtergehäuse zurückbleibt, wobei der absolute Druck an der Entnahmestelle mindestens 0,7 bar ist,
d) Entnahme der filtrierten Flüssigkeit.

2. Verfahren nach Anspruch 1, **dadurch gekennzeichnet, dass** das Innenvolumen des Filtergehäuses mindestens 2 l ist.

3. Verfahren nach Anspruch 1 oder 2, **dadurch gekennzeichnet, dass** mindestens 10 l Überstand filtriert werden.

4. Verfahren nach einem der Ansprüche 1 bis 3, **dadurch gekennzeichnet, dass** die Zellen während des Verfahrens nicht aufgebrochen werden.

5. Verfahren nach einem der Ansprüche 1 bis 4, **dadurch gekennzeichnet, dass** der absolute Druck an der Entnahmestelle mindestens 0,8 bar ist.

6. Verfahren nach einem der Ansprüche 1 bis 5, **dadurch gekennzeichnet, dass** der Differenzdruck mindestens 0,8 bar, vorzugsweise mindestens 1 bar, ist.

7. Verfahren nach einem der Ansprüche 1 bis 6, **dadurch gekennzeichnet, dass** das Filtergehäuse ein Druckgefäß ist, insbesondere ein Druckgefäß ausgelegt für Drücke von mindestens 2 bar, im speziellen bevorzugt von mindestens 5 bar.

8. Verfahren nach einem der Ansprüche 1 bis 7, **dadurch gekennzeichnet, dass** die Temperatur während des Verfahrens zwischen 0° C und 40 °C liegt.

9. Verfahren nach einem der Ansprüche 1 bis 8, **dadurch gekennzeichnet, dass** der Filterkuchen nicht durch zusätzliche Flüssigkeit gewaschen wird und/oder wobei Flüssigkeit aus dem Filterkuchen durch Druckluft ausgebracht wird.

10. Verfahren nach einem der Ansprüche 1 bis 9, weiters umfassend den Schritt: e1) weiteres Filtrieren der in Schritt d) erhaltenen filtrierten Flüssigkeit mit einem zweiten Filter mit einer geringeren Porengröße als der erste Filter, wobei die Porengröße des zweiten Filters im Bereich von 1 µm bis 20 µm ist.

11. Verfahren nach einem der Ansprüche 1 bis 10, weiters umfassend den Schritt: e2) weiteres Filtrieren der in Schritt d) oder e1) erhaltenen filtrierten Flüssigkeit mit einem dritten Filter mit einer geringeren Porengröße als der zweite Filter, wobei die Porengröße des dritten Filters im Bereich von 0,3 µm bis 10 µm ist.

12. Verfahren nach Anspruch 10 oder 11, weiters umfassend den Schritt: f) weiteres Filtrieren der in Schritt e1) oder e2) erhaltenen filtrierten Flüssigkeit mit einem vierten Filter mit einer geringeren Porengröße als der dritte Filter, wobei die Porengröße des dritten Filters im Bereich von 0,05 µm bis 2 µm ist.

13. Verfahren nach einem der Ansprüche 1 bis 12, **dadurch gekennzeichnet, dass** die Zellen pflanzlichen Zellen, speziell Mooszellen, vorzugsweise Laubmooszellen, insbesondere bevorzugt *Physcomitrella patens* Zellen, sind.

14. Verfahren nach einem der Ansprüche 1 bis 13, **dadurch gekennzeichnet, dass** im flüssigen Überstand ein von den Zellen produziertes rekombinantes Protein ist und/oder dass mit der Filtrierung der Flüssigkeit ein rekombinantes Protein des Überstandes gereinigt wird.

15. Verwendung eines Kits mit einem Filtergehäuse wie in einem der Ansprüche 1, 2 oder 7 definiert und mit einem Innenvolumen von mindestens 2 L, wobei das Filtergehäuse ein Druckgefäß mit einem Einlass und einer Luftzufuhröffnung ist, und einem ersten Filter mit einer Porengröße von zwischen 4 µm bis 50 µm, vorzugsweise zusätzlich mit einem zweiten, dritten und/oder vierten Filter wie in einem der Ansprüche 10 bis 12 definiert, in einem Verfahren nach einem der Ansprüche 1 bis 14, insbesondere um Feuchtigkeit aus einem Filtrierkuchen von Zellen zu entfernen.

## Claims

1. Method for separating a liquid supernatant from cells having a cell wall, comprising the steps of:
a) providing a mixture of the cells with a liquid;
b) filling a filter housing with the mixture, wherein a first filter having a pore size of between 4 µm and 50 µm is provided on a flat base surface in the filter housing and the walls of the filter housing are sealingly connected to the flat base surface, which is perforated in a sieve-like manner;
c) applying a differential pressure of at least 0.5 bar to the mixture by means of compressed air, as a result of which the liquid portion of the mixture is pressed through the filter and a filter cake containing cells remains in the filter housing, wherein the absolute pressure at the point of extraction is at least 0.7 bar;
d) extracting the filtered liquid.

2. Method according to claim 1, **characterised in that** the internal volume of the filter housing is at least 2 litres.

3. Method according to either claim 1 or claim 2, **characterised in that** at least 10 litres of supernatant are filtered.

4. Method according to any of claims 1 to 3, **characterised in that** the cells are not broken up during the method.

5. Method according to any of claims 1 to 4, **characterised in that** the absolute pressure at the point of extraction is at least 0.8 bar.

6. Method according to any of claims 1 to 5, **characterised in that** the differential pressure is at least 0.8 bar, preferably at least 1 bar.

7. Method according to any of claims 1 to 6, **characterised in that** the filter housing is a pressure vessel, in particular a pressure vessel designed for pressures of at least 2 bar, particularly preferably of at least 5 bar.

8. Method according to any of claims 1 to 7, **characterised in that** the temperature throughout the method is between 0°C and 40°C.

9. Method according to any of claims 1 to 8, **characterised in that** the filter cake is not washed by means of additional liquid and/or liquid is discharged from the filter cake by means of compressed air.

10. Method according to any of claims 1 to 9, additionally comprising the step of: e1) additionally filtering the filtered liquid obtained in step d) using a second filter having a smaller pore size than the first filter, wherein the pore size of the second filter is in the range of from 1 µm to 20 µm.

11. Method according to any of claims 1 to 10, additionally comprising the step of: e2) additionally filtering the filtered liquid obtained in step d) or e1) using a third filter having a smaller pore size than the second filter, wherein the pore size of the third filter is in the range of from 0.3 to µm to 10 µm.

12. Method according to either claim 10 or claim 11, additionally comprising the step of: f) additionally filtering the filtered liquid obtained in step e1) or e2) using a fourth filter having a smaller pore size than the third filter, wherein the pore size of the third filter is in the range of from 0.05 to µm to 2 µm.

13. Method according to any of claims 1 to 12, **characterised in that** the cells are plant cells, specifically bryophyte cells, preferably moss cells, particularly preferably *Physcomitrella patens* cells.

14. Method according to any of claims 1 to 13, **characterised in that** a recombinant protein produced by the cells is present in the liquid supernatant and/or **in that**, by filtering the liquid, a recombinant protein of the supernatant is purified.

15. Use of a kit comprising a filter housing as defined in any of claims 1, 2 or 7 and having an internal volume of at least 2 litres, wherein the filter housing is a pressure vessel having an inlet and an air supply opening, and comprising a first filter having a pore size of between 4 µm and 50 µm, preferably additionally comprising a second, third and/or fourth filter as defined in any of claims 10 to 12, in a method according to any of claims 1 to 14, in particular to extract moisture from a filter cake of cells.

## Revendications

1. Procédé servant à séparer un surnageant liquide de cellules avec une paroi cellulaire, comprenant les étapes :
a) de fourniture d'un mélange des cellules avec un liquide,
b) de remplissage d'un boîtier filtrant avec le mélange, dans lequel un premier filtre avec une grandeur de pores entre 4 µm à 50 µm est prévu sur une surface de fond plate dans le boîtier filtrant et les parois du boîtier filtrant sont reliées de manière étanche à la surface de fond plate ajourée à la manière d'un crible,
c) d'application d'une pression différentielle d'au moins 0,5 bar au niveau du mélange par air comprimé, moyennant quoi la fraction de liquide du mélange à travers le filtre est pressée et il reste un gâteau de filtre contenant des cellules dans le boîtier filtrant, dans lequel la pression absolue au niveau de l'emplacement de prélèvement est d'au moins 0,7 bar,
d) de prélèvement du liquide filtré.

2. Procédé selon la revendication 1, **caractérisé en ce que** le volume intérieur du boîtier filtrant est d'au moins 2 l.

3. Procédé selon la revendication 1 ou 2, **caractérisé en ce qu'**au moins 10 l de surnageant sont filtrés.

4. Procédé selon l'une quelconque de revendications 1 à 3, **caractérisé en ce que** les cellules ne sont pas brisées pendant le procédé.

5. Procédé selon l'une quelconque des revendications 1 à 4, **caractérisé en ce que** la pression absolue au niveau de l'emplacement de prélèvement est d'au moins 0,8 bar.

6. Procédé selon l'une quelconque des revendications 1 à 5, **caractérisé en ce que** la pression différentielle est d'au moins 0,8 bar, de préférence d'au moins 1 bar.

7. Procédé selon l'une quelconque des revendications 1 à 6, **caractérisé en ce que** le boîtier filtrant est un récipient sous pression, en particulier un récipient sous pression configuré pour des pressions d'au moins 2 bar, spécifiquement de manière préférée d'au moins 5 bar.

8. Procédé selon l'une quelconque des revendications 1 à 7, **caractérisé en ce que** la température est comprise entre 0 °C et 40 °C pendant le procédé.

9. Procédé selon l'une quelconque des revendications 1 à 8, **caractérisé en ce que** le gâteau de filtre n'est pas lavé par du liquide supplémentaire, et/ou dans lequel du liquide est déversé hors du gâteau de filtre du fait de l'air comprimé.

10. Procédé selon l'une quelconque des revendications 1 à 9, comprenant en outre l'étape : e1) de filtration ultérieure du liquide filtré obtenu à l'étape d) avec un deuxième filtre avec une grandeur de pores inférieure à celle du premier filtre, dans lequel la grandeur de pores du deuxième filtre se situe dans la plage allant de 1 µm à 20 µm.

11. Procédé selon l'une quelconque des revendications 1 à 10, comprenant en outre l'étape : e2) de filtration ultérieure du liquide filtré obtenu à l'étape d) ou e1) avec un troisième filtre avec une grandeur de pores inférieure à celle du deuxième filtre, dans lequel la grandeur de pores du troisième filtre se situe dans la plage allant de 0,3 µm à 10 µm.

12. Procédé selon la revendication 10 ou 11, comprenant en outre l'étape : f) de filtration ultérieure du liquide filtré obtenu à l'étape e1) ou e2) avec un quatrième filtre avec une grandeur de pores inférieure à celle du troisième filtre, dans lequel la grandeur de pores du troisième filtre se situe dans la plage allant de 0,05 µm à 2 µm.

13. Procédé selon l'une quelconque des revendications 1 à 12, **caractérisé en ce que** les cellules sont des cellules végétales, de manière spécifique des cellules de bryophyte, de préférence des cellules de mousse, en particulier de manière préférée des cellules de *Physcomitrella patens.*

14. Procédé selon l'une quelconque des revendications 1 à 13, **caractérisé en ce qu'**une protéine recombinante produite par les cellules est dans le surnageant liquide, et/ou qu'une protéine recombinante du surnageant est nettoyée avec la filtration du liquide.

15. Utilisation d'un kit avec un boîtier filtrant tel que défini dans l'une quelconque des revendications 1, 2 ou 7 et avec un volume intérieur d'au moins 2 l, dans laquelle le boîtier filtrant est un récipient sous pression avec une admission et une ouverture d'arrivée d'air, et un premier filtre avec une grandeur de pores entre 4 µm à 50 µm, de préférence en outre avec un deuxième, troisième et/ou quatrième filtre tel que défini dans l'une quelconque des revendications 10 à 12, dans un procédé selon l'une quelconque des revendications 1 à 14, en particulier pour supprimer de l'humidité provenant d'un gâteau de filtration de cellules.
